# EUROPEAN PATENT SPECIFICATION

(11) **EP 4 250 432 B1**
(45) Date of publication and mention of the grant of the patent: **29.04.2026**
(21) Application number: 22898785.5
(22) Date of filing: 06.09.2022
(51) Int. Cl.: H01M 10/42, H01M 10/48, G01N 33/00

(54) **BATTERY MODULE AND BATTERY PACK INCLUDING THE SAME**
BATTERIEMODUL UND BATTERIEPACK DAMIT
MODULE DE BATTERIE ET BLOC-BATTERIE COMPRENANT CELUI-CI

(30) Priority: 24.11.2021 KR 20210163384
(43) Date of publication of application: 27.09.2023
(73) Proprietor: LG Energy Solution, Ltd., Seoul 07335 (KR)
(72) Inventor: KIM, Dooseung, Daejeon 34122 (KR); LEE, Jung Hoon, Daejeon 34122 (KR); KIM, Seho, Daejeon 34122 (KR); PARK, Jeong Gi, Daejeon 34122 (KR)
(74) Representative: Goddar, Heinz J.
(86) International application number: PCT/KR2022/013343
(87) International publication number: WO 2023/096101

(56) References cited:
- JP-A- 2008 103 283
- JP-A- 2020 202 104
- JP-B2- 7 222 818
- KR-A- 20130 132 500
- KR-A- 20140 064 176
- KR-A- 20210 108 209
- KR-B1- 101 155 134
- KR-B1- 101 225 236
- KR-B1- 102 172 960
- US-A1- 2021 265 699

## Description

### [TECHNICAL FIELD]

### Cross Citation with Related Application(s)

This application claims the benefit of Korean Patent Application No. 10-2021-0163384 filed on November 24, 2021 with the Korean Intellectual Property Office.

The present disclosure relates to a battery module and a battery pack including the same, and more particularly to a battery module having enhanced safety and a battery pack including the same.

### [BACKGROUND]

With the technology development and increased demand for mobile devices, demand for secondary batteries as energy sources has been rapidly increasing. In particular, a secondary battery has attracted considerable attention as an energy source for power-driven devices, such as an electric bicycle, an electric vehicle, and a hybrid electric vehicle, as well as an energy source for mobile devices, such as a mobile phone, a digital camera, a laptop computer and a wearable device.

In small mobile devices, one, or two, or three battery cells are used per device, while middle or large-sized devices such as vehicles require high power and large capacity. Therefore, a middle or large-sized battery module having a plurality of battery cells electrically connected to one another is used.

Since middle or large-sized battery modules are preferably manufactured with as small a size and weight as possible, a prismatic battery, a pouch-type battery, or the like, which can be stacked with high integration and has a small weight relative to capacity, is mainly used as a battery cell of the middle or large-sized battery modules. Such a battery module has a structure in which a plurality of cell assemblies including a plurality of unit battery cells are connected in series to obtain high output. And, the battery cell includes positive and negative electrode current collectors, a separator, an active material, an electrolyte, and the like, and thus can be repeatedly charged and discharged through an electrochemical reaction between components.

At this time, an all-solid-state battery using the above-mentioned electrolyte as an all-solid material is being developed, but the all-solid-state battery is a battery that utilizes a solid electrolyte instead of the liquid electrolyte, and has an advantage of having higher thermal stability than the existing lithium secondary battery using a liquid electrolyte. In addition, the all-solid-state battery is more advantageous than a conventional lithium secondary battery in terms of high energy density and output characteristics, simplification of the manufacturing process and enlargement/compactness of a battery. Thus, recently, research and interest have been focused on these points.

Particularly, among all-solid-state batteries, a sulfide-based all-solid-state battery using a sulfide-based electrolyte has the characteristics of being relatively inexpensive and safe, but when the sulfide-based electrolyte is exposed to moisture, there is a risk of generating hydrogen sulfide (H₂S), which is a hazardous material.

Therefore, even when hydrogen sulfide is generated, there is a need for a new technology that can neutralize hydrogen sulfide so that hydrogen sulfide does not leak outside the battery module and the battery pack.

KR 102 172 960 B1 describes sensing of gas in DC power supplies.

US 2021 / 265 699 A1 describes sensing of gas that is generated as a result of a reaction between a liquid electrolyte and an electrode.

JP 2008 103 283 A describes neutralization of gas that is generated as a result of a reaction between a solid electrolyte and moisture.

JP2020202104 A describes neutralization of hydrogen sulfide gas by opening a packaging material containing an adsorbent upon detection of the gas by a hydrogen sulfide sensor.

### [DETAILED DESCRIPTION OF THE INVENTION]

### [Technical Problem]

It is an object of the present disclosure to provide a battery module having enhanced safety, and a battery pack including the same.

However, the objects of the present disclosure are not limited to the aforementioned objects, and other objects which are not mentioned herein should be clearly understood by those skilled in the art from the following detailed description and the accompanying drawings.

### [Technical Solution]

According to one embodiment of the present disclosure, there is provided a battery module comprising: a battery cell stack in which a plurality of battery cells are stacked, a module frame that surrounds the battery cell stack, a bus bar frame that covers the portion of the battery cell stack exposed from the module frame, a bus bar that is connected to an electrode lead protruding from the battery cell stack through the bus bar frame, a gas sensor formed on the bus bar frame and configured to detect a leakage of hydrogen sulfide, a bus bar cover portion that covers the bus bar, and a neutralizer housing portion formed on the bus bar cover portion, wherein a neutralizer is formed inside the neutralizer housing portion, and the neutralizer housing portion is configured to be opened to inject a neutralizer responsive to the leakage.

The gas sensor may be formed to be adjacent to the bus bar.

The bus bar comprises a terminal bus bar, and the gas sensor may be formed to be adjacent to the terminal of the terminal bus bar.

The bus bar cover portion may cover the terminal of the terminal bus bar.

The battery module according to another embodiment of the present disclosure further comprises an end plate that covers the bus bar frame, and may further comprise a gas sensor formed on the end plate.

The battery module according to yet another embodiment of the present disclosure further comprises a gas sensor formed between an upper part of the battery cell stack and the module frame.

The neutralizer may comprise at least one component selected from the group comprising an iron compound and a catalyst.

The neutralizer housing portion may comprise a bulb.

The battery module may further comprise a neutralizer injection portion formed in the neutralizer housing portion.

According to yet another embodiment of the present disclosure, there is provided a battery pack comprising the above-mentioned battery module.

### [Advantageous Effects]

A battery module according to one embodiment of the present disclosure includes a gas sensor that can detect leakage of hydrogen sulfide and a neutralizer housing portion that can neutralize hydrogen sulfide, whereby during leakage of hydrogen sulfide, it is possible to detect and neutralize this to improve the safety of the battery module and battery pack.

The effects of the present disclosure are not limited to the effects mentioned above and additional other effects not described above will be clearly understood from the description of the appended claims by those skilled in the art.

### [BRIEF DESCRIPTION OF THE DRAWINGS]

Fig. 1 is a perspective view showing a battery module according to one embodiment of the present disclosure;
Fig. 2 is a perspective view showing a state in which all the components of Fig. 1 are combined;
Fig. 3 is an exploded perspective view showing some of the components of Fig. 1;
Fig. 4 is an exploded perspective view showing some of the components of the battery module according to another embodiment of the present disclosure;
Fig. 5 is a diagram showing a battery module according to another embodiment of the present disclosure.
Fig. 6 is a perspective view showing a bus bar cover portion and a neutralizer housing portion included in the battery module of the present disclosure;
Fig. 7 is a perspective view showing another shape of a bus bar cover portion and a neutralizer housing portion included in the battery module of the present disclosure;
Fig. 8 is a perspective view showing a battery cell included in the battery module of the present disclosure; and
Fig. 9 is a schematic diagram of a hydrogen sulfide neutralization system operating in a battery pack and a device including the battery pack according to yet another embodiment of the present disclosure.

### [DETAILED DESCRIPTION OF THE EMBODIMENTS]

Hereinafter, various embodiments of the present disclosure will be described in detail with reference to the accompanying drawings so that those skilled in the art can easily carry out them. The present disclosure may be modified in various different ways, and is not limited to the embodiments set forth herein.

Portions that are irrelevant to the description will be omitted to clearly describe the present disclosure, and like reference numerals designate like elements throughout the description.

Further, in the drawings, the size and thickness of each element are arbitrarily illustrated for convenience of description, and the present disclosure is not necessarily limited to those illustrated in the drawings. In the drawings, the thickness of layers, regions, etc. are exaggerated for clarity. In the drawings, for convenience of description, the thicknesses of a part and an area are exaggeratedly illustrated.

Further, it will be understood that when an element such as a layer, film, region, or plate is referred to as being "on" or "above" another element, it can be directly on the other element or intervening elements may also be present. In contrast, when an element is referred to as being "directly on" another element, it means that other intervening elements are not present. Further, a certain part being located "above" or "on" a reference portion means the certain part being located above or below the reference portion and does not particularly mean the certain part "above" or "on" toward an opposite direction of gravity.

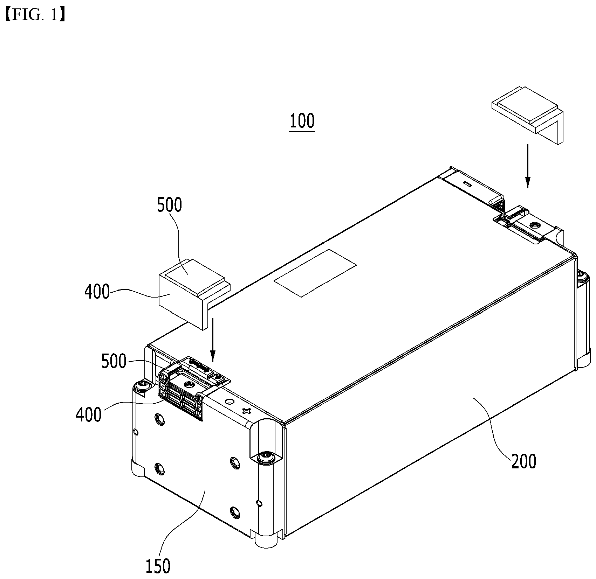

Hereinafter, a battery module of the present disclosure will be described with reference to Figs. 1 to 3 and Figs. 6 to 8.

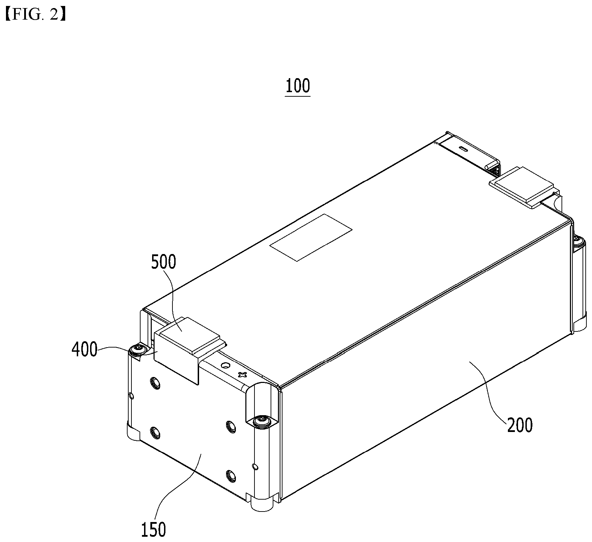

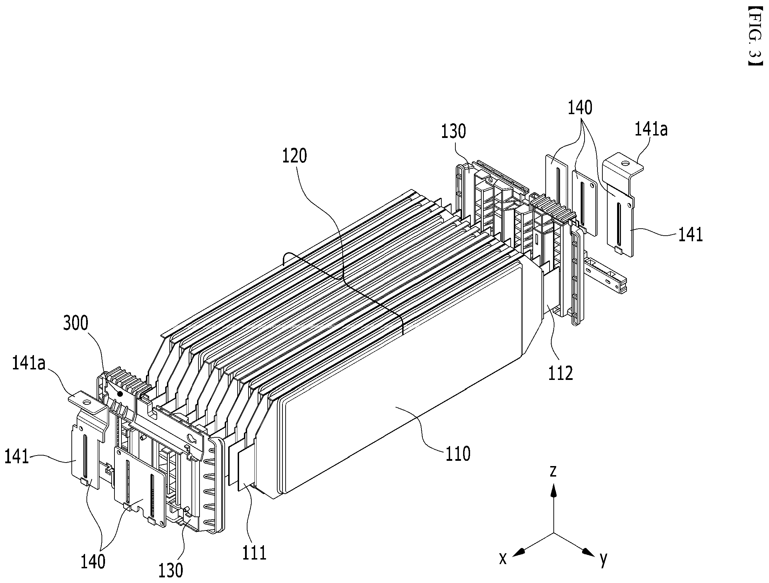

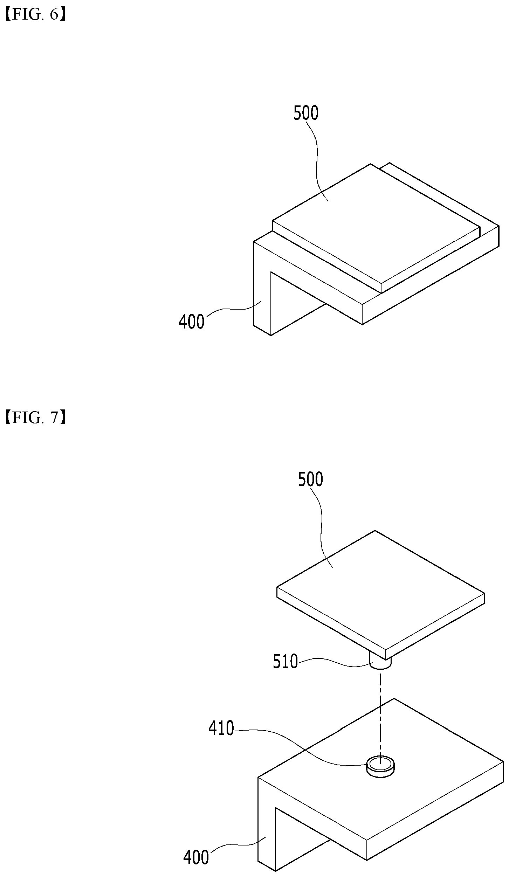

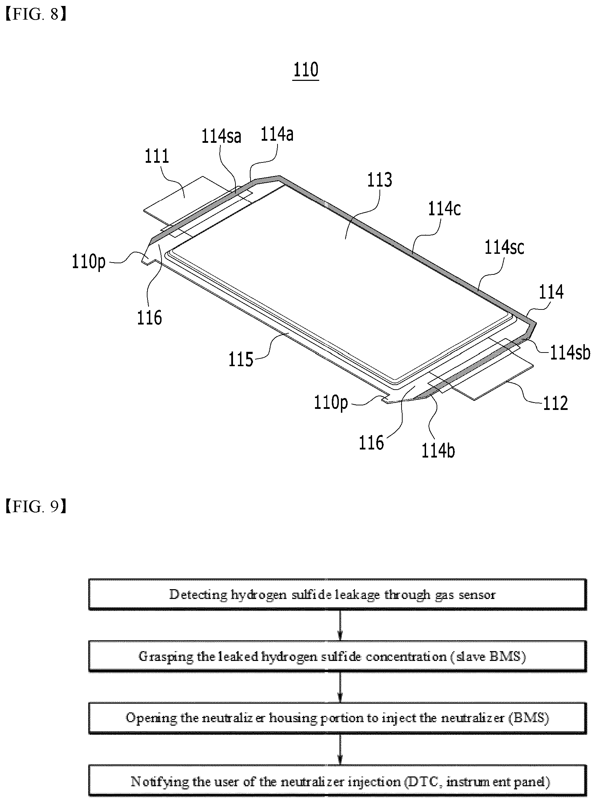

Fig. 1 is a perspective view showing a battery module according to one embodiment of the present disclosure. Fig. 2 is a perspective view showing a state in which all the components of Fig. 1 are combined. Fig. 3 is an exploded perspective view showing some of the components of Fig. 1. Fig. 6 is a perspective view showing a bus bar cover portion and a neutralizer housing portion included in the battery module of the present disclosure. Fig. 7 is a perspective view showing another shape of a bus bar cover portion and a neutralizer housing portion included in the battery module of the present disclosure. Fig. 8 is a perspective view showing a battery cell included in the battery module of the present disclosure.

Referring to Figs. 1 to 3, a battery module 100 according to the present embodiment includes a battery cell stack 120 in which a plurality of battery cells 110 are stacked, and a module frame 200 that surrounds the battery cell stack 120.

First, the battery cell 110 is preferably a pouch-type battery cell, and can be formed in a rectangular sheet-like structure. For example, referring to Fig. 8, the battery cell 110 according to the present embodiment has a structure in which two electrode leads 111 and 112 face each other and protrude from one end part 114a and the other end part 114b of the cell main body 113, respectively. That is, the battery cell 110 includes electrode leads 111 and 112 that are protruded in mutually opposite directions. More specifically, the electrode leads 111 and 112 are connected to an electrode assembly (not shown), and are protruded from the electrode assembly (not shown) to the outside of the battery cell 110. However, unlike those shown in Fig. 8, the two electrode leads 111 and 112 may have a structure protruding from one side surface of the same battery cell 110. Therefore, the two electrode leads 111 and 112 may protrude in the same direction.

Meanwhile, the battery cell 110 can be produced by joining both end parts 114a and 114b of a cell case 114 and one side part 114c connecting them in a state in which an electrode assembly (not shown) is housed in a cell case 114. In other words, the battery cell 110 according to the present embodiment has a total of three sealing parts 114sa, 114sb and 114sc, wherein the sealing parts 114sa, 114sb and 114sc have a structure that is sealed by a method such as heat-sealing, and the remaining other side part may be composed of a connection part 115. In particular, the sealing parts 114sa, 114sb and 114sc may include a sealing part 114sc formed in the longitudinal direction of the battery cell, and a sealing parts 114sa and 114sb formed in the width direction of the battery cell. In addition, the battery cell may have four sealing parts by further including one sealing part formed in the longitudinal direction of the battery cell together with the three sealing parts 114sa, 114sb and 114sc. The cell case 114 may be formed of a laminated sheet including a resin layer and a metal layer. At this time, between both ends 114a and 114b of the battery case 114 may be defined as the longitudinal direction of the battery cell 110, and between one side part 114c connecting both ends 114a and 114b of the battery case 114 and the connection part 115 may be defined as a width direction of the battery cell 110.

Further, the connection part 115 may extend long along one edge of the battery cell 110, and a bat-ear 110p may be formed at an end of the connection part 115. Further, while the cell case 114 is sealed with the protruding electrode leads 111 and 112 being interposed therebetween, a terrace part 116 may be formed between the electrode leads 111 and 112 and the cell main body 113. That is, the battery cell 110 may include a terrace part 116 formed to extend from the cell case 114 in the direction in which the electrode leads 111 and 112 protrude.

Such a battery cell 110 may be composed by a plurality of numbers, and the plurality of battery cells 110 may be stacked so as to be electrically connected to each other, thereby forming a battery cell stack 120. Particularly, as shown in Fig. 3, a plurality of battery cells 110 may be stacked along the direction parallel to the y-axis. Thereby, the electrode leads 111 and 112 may protrude in the x-axis direction and the -x-axis direction, respectively. However, as described above, in the battery cell 110 in which the electrode leads 111 and 112 protrude in the same direction, the two electrode leads 111 and 112 may protrude equally in the x-axis direction or in the -x-axis direction.

Referring to Figs. 1 to 3 again, the module frames 200 may be a mono frame that surrounds the battery cell stack 120 excluding the front and rear surfaces thereof. However, the module frame 200 is not limited thereto, and may include a module frame structure that includes an L-shaped frame or a U-shaped frame which is opened in the upper surface, the front surface and the rear surface thereof and covers the lower part and both side parts of the battery cell stack 120, and an upper plate that covers an upper part of the battery cell stack 120. The battery cell stack 120 housed inside the module frame 200 can be physically protected through the module frame 200.

In addition, although not shown in the figure, the battery module 100 according to the present embodiment may further include a thermal conductive resin layer located between the lower surface of the battery cell stack 120 and the bottom part of the module frame 200, and the thermal conductive resin layer can play a role of transferring heat generated in the battery cell 110 to the bottom of the battery module 100 and fixing the battery cell stack 120. In addition, together with the thermal conductive resin layer, a cooling flow path or an additional heat dissipation material formed to contact the lower surface of the battery cell stack 120 is formed, thereby enabling surface cooling of the battery cell stack 120.

The end plate 150 may cover the front and rear surfaces of the battery cell stack 120 that are opened in the module frame 200. The end plate 150 may be weld-coupled to the front and rear end edges of the module frame 200.

A bus bar frame 130 may be formed between the end plate 150 and the front and rear surfaces of the battery cell stack 120. That is, the end plate 150 may cover the bus bar frame 130. At this time, the bus bar frame 130 may cover the portion of the battery cell stack 120 exposed from the module frame 200.

The plurality of bus bars 140 mounted to the bus bar frame 130 are formed to protrude from the battery cells 110 through the bus bar frame 130, and can be connected with the electrode leads 111 and 112 mounted to the bus bar frame 130. At this time, the bus bar 140 may include a terminal bus bar 141, and the terminal bus bar 141 may include a terminal 141a.

In addition, the battery module according to the present embodiment may include a bus bar cover portion 400 that covers the portion where the bus bar 140, particularly the terminal bus bar 141, is exposed to the outside of the end plate 150. Specifically, the bus bar cover portion 400 is formed so as to cover the terminal 141a of the terminal bus bar 141, so that the terminal 141a can be prevented from being exposed to the outside of the battery module 100. Further, as will be described later, it can play a role of temporarily preventing leakage of hydrogen sulfide, which is highly likely to flow out from the terminal 141a.

Among all-solid-state batteries using sulfide-based electrolyte and all-solid-state batteries using electrolyte as an all-solid material, a sulfide-based all-solid-state battery using a sulfide-based electrolyte has a risk of generating hydrogen sulfide (H₂S), which is a hazardous material, when the sulfide-based electrolyte is exposed to moisture.

Therefore, there is a need for a structure that can neutralize hydrogen sulfide so that hydrogen sulfide does not leak to the outside of the battery module and battery pack even if hydrogen sulfide is generated.

Therefore, referring to Figs. 1 to 3, the battery module according to the present embodiment includes a gas sensor 300 formed on the bus bar frame 130, a bus bar cover portion 400 covering the bus bar 140, and a neutralizer housing portion 500 formed on the bus bar cover portion 400.

Specifically, referring to Fig. 3, the gas sensor 300 according to the present embodiment may be formed on the bus bar frame 130 so as to be adjacent to the bus bar 140.

Generally, if hydrogen sulfide leaks, the hydrogen sulfide is highly likely to leak through the terminal 141a of the battery module 100. Therefore, it is necessary to immediately grasp hydrogen sulfide leakage by forming a structure capable of detecting hydrogen sulfide to be adjacent to the terminal 141a.

Therefore, the gas sensor 300 according to the present embodiment may be formed to be adjacent to the bus bar 140. More specifically, the gas sensor 300 is formed to be adjacent to the terminal 141a of the terminal bus bar 141, so that the effect of immediately detecting hydrogen sulfide leakage can be achieved.

At this time, after the gas sensor 300 detects the leakage of hydrogen sulfide, it is necessary to form a structure for neutralizing hydrogen sulfide in order to immediately remove hydrogen sulfide.

Therefore, referring to Fig. 6, the battery module 100 according to the present embodiment includes the neutralizer housing portion 500 formed on the bus bar cover portion 400 as described above.

The neutralizer housing portion 500 may be formed to be adjacent to the terminal 141a of the terminal bus bar 141 with a high risk of hydrogen sulfide leakage in order to immediately remove the hydrogen sulfide after the gas sensor 300 detects the leakage of hydrogen sulfide.

Therefore, the neutralizer housing portion 500 may be formed on the bus bar cover portion 400 that covers the terminal 141a of the terminal bus bar 141.

Meanwhile, the neutralizer housing portion 500 may include a neutralizer formed inside the neutralizer housing portion 500.

At this time, the neutralizer may include at least one component selected from the group comprising iron compounds and catalysts. The iron compound may include ferric sulfate (Fe₂(SO₄)₃), iron(III) oxide (Fe₂O₃), iron oxyhydroxide (FeO(OH)) and/or iron citrate (FeC₆H₅O₇). The catalyst may include iron hydroxide (Fe(OH)₂, Fe(OH)₃) and/or zinc hydroxide. Therefore, the neutralizer may include one or more components selected from the group comprising the iron compound and the catalyst, and hydrogen sulfide can be neutralized through the above component.

In examples not according to the claimed invention, the neutralizer housing portion 500 may include at least one kind of shapes selected from the group comprising a pad, and a pocket.

Specifically, when the neutralizer housing portion 500 includes the pad shape the neutralizer is stored in the form of microcapsules inside the pad shape, and hydrogen sulfide is neutralized by the neutralizer. Specifically, the pad-shaped neutralizer housing portion 500 allows hydrogen sulfide and the neutralizer to react inside the pad while hydrogen sulfide passes through the pad.

Further, the pocket shape may form a pocket of a polymeric material and/or a metallic material, and house the neutralizer therein. Therefore, during generation of hydrogen sulfide, the pocket is crushed and the neutralizer leaks to the outside to react with hydrogen sulfide.

At this time, the pad shape and the pocket shape may be formed on the bus bar cover portion 400 as shown in Fig. 6.

Meanwhile, referring to Fig. 7, the neutralizer housing portion 500 according to the invention, which may include an aerosol fire extinguisher shape, injects the neutralizer into surrounding air, in particular in the form of an aerosol. Specifically, as shown in Fig. 7, the aerosol fire extinguisher-shaped neutralizer housing portion 500 may include a neutralizer injection portion 510 formed under the neutralizer housing portion 500. Moreover, a hole 410 may be formed on the bus bar cover 400 to minimize the protruding portion of the neutralizer injection portion 510. At this time, the neutralizer injection portion 510 can be formed to be coupled to the hole 410, and when hydrogen sulfide flows out, the neutralizer may be injected over a wide area through the neutralizer injection portion 510 to neutralize hydrogen sulfide.

Further, the bulb shape may specifically include a plastic bulb shape. The bulb shape may include a neutralizer housing portion 500 and a neutralizer injection portion 510 as shown in Fig. 7, and in some cases, it may further include a liquid formed in the neutralizer injection portion 510. Therefore, when the battery module 100 generates heat, a liquid may evaporate to open the neutralizer injection portion 510 to neutralize hydrogen sulfide.

At this time, the neutralizer housing portion 500 including the above shape, that is, the pad, pocket, aerosol, and/or bulb shape, may be formed into the shape of a rectangular parallelepiped or a hexahedron with rounded corners so as to be formed on the bus bar cover portion 400.

Further, the neutralizer housing portion 500 of the present disclosure can be formed in various shapes within a range that can secure the neutralization performance of the neutralizer housing portion.

Therefore, the neutralizer housing portion 500 includes the above shape, and the neutralizer is housed inside the neutralizer housing portion 500, whereby when leakage of hydrogen sulfide is detected by a gas sensor 300, the neutralizer housing portion 500 is opened to inject the neutralizer, thereby neutralizing hydrogen sulfide. Further, by securing a structure that can neutralize hydrogen sulfide, which is a hazardous material, when it leaks, the safety of the battery module 100, the battery pack including the battery module 100 and the device can be improved.

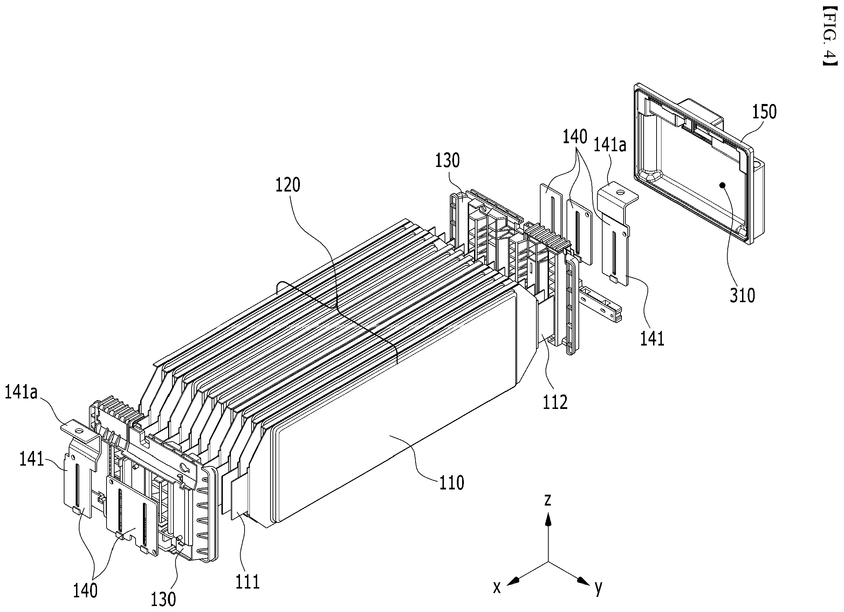

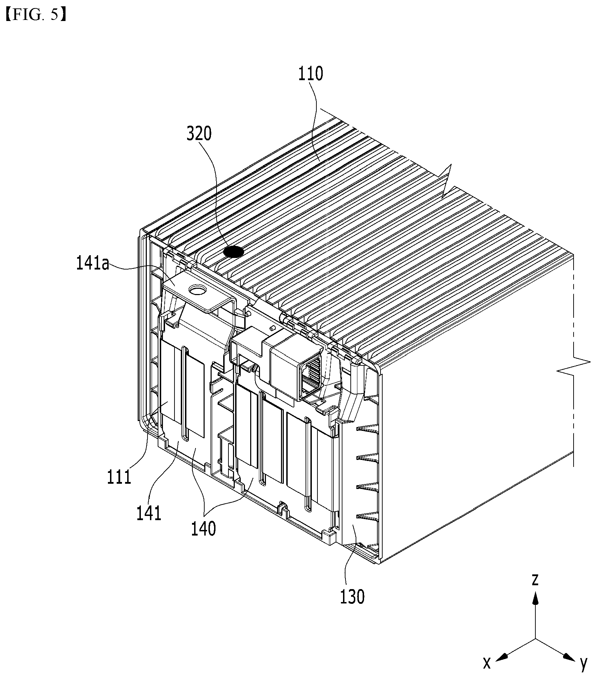

Next, a battery module according to modified embodiments of the present disclosure will be described with reference to Figs. 4 and 5. This embodiment may include all contents related to the battery module described above, and only the contents that do not overlap with those described above will be described.

Fig. 4 is an exploded perspective view showing some of the components of the battery module according to another embodiment of the present disclosure. Fig. 5 is a diagram showing a battery module according to another embodiment of the present disclosure.

Referring to Fig. 4, the battery module 100 according to the present embodiment may further include a gas sensor 310 formed on the end plate 150. The gas sensor 310 may be formed to be adjacent to the terminal 141a of the terminal bus bar 141. Therefore, the gas sensor 310 is formed on the inner surface or the outer surface of the end plate 150 adjacent to the terminal 141a, so that generation and leakage of hydrogen sulfide can be immediately detected through the gas sensor 310.

Referring to Fig. 5, the battery module according to the present embodiment may further include a gas sensor 320 that is formed between an upper part of the battery cell stack 120 and the module frame 200. The gas sensor 320 may be formed between the upper part of the battery cell stack 120 adj acent to the terminal 141a of the terminal bus bar 141 and the upper part of the module frame 200.

In particular, the gas sensor 320 may be formed on the inner surface of the upper part of the module frame 200, and may be formed on the upper part of the battery cell stack 120.

Leakage of hydrogen sulfide can be detected through the gas sensor 320 formed at the position to immediately neutralize hydrogen sulfide.

Further, based on the above contents, the gas sensors 300, 310 and 320 according to embodiments of the present disclosure can be formed in at least one position selected from between the bus bar frame 130 and the terminal 141a, between the end plate 150 and the terminal 141a, and between the battery cell stack 120 and the module frame 200. Therefore, not only a case where the gas sensors are formed in all positions where the above-mentioned gas sensors 300, 310 and 320 can be formed, but also a case where a gas sensor is formed in a part of the formable positions can be included in the scope of the present disclosure.

In addition, one or a plurality of gas sensors 300 are formed to be adjacent to the terminal 141a of the bus bar 140, particularly, the terminal bus bar 141 as described above, so that leakage of hydrogen sulfide can be detected. Further, the neutralizer housing portion 500 is formed to be adj acent to the leakage position of the hydrogen sulfide, thereby detecting the leakage of hydrogen sulfide and at the same time, injecting the neutralizer to neutralize hydrogen sulfide. Therefore, it is possible to prevent the safety of the battery module 100 from being deteriorated due to the leakage of hydrogen sulfide, and furthermore, to secure the safety of the battery pack and the device in which the battery module 100 is arranged.

A battery pack according to yet another embodiment of the present invention will be described below.

The battery pack according to the present embodiment includes the battery module described above. In addition, the battery pack of the present disclosure may have a structure in which one or more of the battery modules according to the present embodiment are gathered, and packed together with a battery management system (BMS) and a cooling device that control and manage battery's temperature, voltage, etc.

The battery pack can be applied to various devices. Such a device can be applied to a vehicle means such as an electric bicycle, an electric vehicle, or a hybrid vehicle, but the present disclosure is not limited thereto, and is applicable to various devices that can use a battery module, which also falls under the scope of the present disclosure.

Fig. 9 is a schematic diagram of a hydrogen sulfide neutralization system operating in a battery pack and a device including the battery pack according to yet another embodiment of the present disclosure.

Referring to Fig. 9, the battery pack and device including the battery module of the present disclosure includes a step of detecting hydrogen sulfide leakage through a gas sensor, and a step of grasping the leaked hydrogen sulfide concentration. At this time, after grasping the leaked hydrogen sulfide concentration (slave BMS), a step of opening the neutralizer housing portion 500 to inject the neutralizer (BMS) is included. In addition, after the neutralizer is injected as described above, a step of notifying the user of the neutralizer injection (DTC, instrument panel) may be further included.

Therefore, the gas sensors 300 , 310 and 320 and the neutralizer housing portion 500 included in the battery module 100 described in the present embodiments can constitute a neutralization system of hydrogen sulfide through the configuration of the slave BMS, BMS and instrument panel included in the battery pack and device. That is, the battery pack and device according to the present embodiment include a hydrogen sulfide neutralization system comprising the steps of grasping the degree of leakage of hydrogen sulfide detected through the gas sensor, opening the neutralizer housing portion 500, and notifying the user of this, thereby capable of securing more improved safety.

Although preferred embodiments of the present disclosure have been described in detail above, the scope of the present disclosure is not limited thereto, and numerous other modifications and embodiments can be devised by those skilled in the art, without departing from the scope of the invention defined in the appended claims.

### [Description of Reference Numerals]

100: battery module
110: battery cell
120: battery cell stack
130: bus bar frame
140: bus bar
141: terminal bus bar
150: end plate
200: module frame
300: gas sensor
400: bus bar cover portion
500: neutralizer housing portion

## Claims

1. A battery module (100) comprising:
a battery cell stack (120) in which a plurality of battery cells (110) are stacked,
a module frame (200) that surrounds the battery cell stack (120),
a bus bar frame (130) that covers the portion of the battery cell stack (120) exposed from the module frame (200),
a bus bar (140) that is connected to an electrode lead (111, 112) protruding from the battery cell stack (120) through the bus bar frame (130),
a gas sensor (300) formed on the bus bar frame (130),
and a bus bar cover portion (400) that covers the bus bar (140),
**characterized in that** the gas sensor (300) is configured to detect a leakage of hydrogen sulfide, and **in that** the battery module (100) comprises:
a neutralizer housing portion (500) formed on the bus bar cover portion (400),
wherein a neutralizer is formed inside the neutralizer housing portion (500),
and the neutralizer housing portion (500) is configured to be opened to discharge the neutralizer responsive to the leakage.

2. The battery module (100) according to claim 1 wherein:
the gas sensor (300) is formed to be adjacent to the bus bar (140).

3. The battery module (100) according to claim 2 wherein:
the bus bar (140) comprises a terminal bus bar (141), and
the gas sensor (300) is formed to be adjacent to the terminal (141a) of the terminal bus bar (141).

4. The battery module (100) according to claim 3 wherein:
the bus bar cover portion (400) covers the terminal (141a) of the terminal bus bar (141).

5. The battery module (100) according to claim 1 wherein:
the battery module (100) further comprises an end plate (150) that covers the bus bar frame (130), and
further comprises a gas sensor (310) formed on the end plate (150).

6. The battery module (100) according to claim 1, further comprising:
a gas sensor (320) formed between an upper part of the battery cell stack (120) and the module frame (200).

7. The battery module (100) according to claim 1 wherein:
the neutralizer comprises at least one component selected from the group comprising an iron compound and a catalyst.

8. The battery module (100) according to claim 1 wherein:
the neutralizer housing portion (500) comprises a bulb.

9. The battery module (100) according to claim 1, further comprising:
a neutralizer injection portion (510) formed in the neutralizer housing portion (500).

10. A battery pack comprising the battery module (100) as set forth in claim 1.

## Patentansprüche

1. Batteriemodul (100) aufweisend:
einen Batteriezellenstapel (120), in dem mehrere Batteriezellen (110) gestapelt sind,
einen Modulrahmen (200), der den Batteriezellenstapel (120) umgibt,
einen Sammelschienenrahmen (130), der den Abschnitt des Batteriezellenstapels (120) bedeckt, der außerhalb des Modulrahmens (200) freiliegt,
eine Sammelschiene (140), die mit einer Elektrodenleitung (111, 112) verbunden ist, die von dem Batteriezellenstapel (120) durch den Sammelschienenrahmen (130) vorsteht,
einen Gassensor (300), der auf dem Sammelschienenrahmen (130) ausgebildet ist,
und einen Sammelschienenabdeckungsabschnitt (400), der die Sammelschiene (140) bedeckt,
**dadurch gekennzeichnet, dass** der Gassensor (300) konfiguriert ist, um eine Leckage von Schwefelwasserstoff zu erfassen, und dadurch, dass das Batteriemodul (100) aufweist:
einen Neutralisatorgehäuseabschnitt (500), der auf dem Sammelschienenabdeckungsabschnitt (400) ausgebildet ist,
wobei ein Neutralisator innerhalb des Neutralisatorgehäuseabschnitts (500) ausgebildet ist,
und der Neutralisatorgehäuseabschnitt (500) konfiguriert ist, um geöffnet zu werden, um den Neutralisator als Reaktion auf die Leckage zu entladen.

2. Batteriemodul (100) nach Anspruch 1, wobei:
der Gassensor (300) benachbart zu der Sammelschiene (140) ausgebildet ist.

3. Batteriemodul (100) nach Anspruch 2, wobei:
die Sammelschiene (140) eine Anschlusssammelschiene (141) aufweist, und
der Gassensor (300) benachbart zu dem Anschluss (141a) der Anschlusssammelschiene (141) ausgebildet ist.

4. Batteriemodul (100) nach Anspruch 3, wobei:
der Sammelschienenabdeckungsabschnitt (400) den Anschluss (141a) der Anschlusssammelschiene (141) bedeckt.

5. Batteriemodul (100) nach Anspruch 1, wobei:
das Batteriemodul (100) ferner eine Endplatte (150) aufweist, die den Sammelschienenrahmen (130) bedeckt, und
ferner einen Gassensor (310) aufweist, der auf der Endplatte (150) ausgebildet ist.

6. Batteriemodul (100) nach Anspruch 1, ferner aufweisend:
einen Gassensor (320), der zwischen einem oberen Teil des Batteriezellenstapels (120) und dem Modulrahmen (200) ausgebildet ist.

7. Batteriemodul (100) nach Anspruch 1, wobei:
der Neutralisator mindestens eine Komponente aufweist, die aus der Gruppe ausgewählt ist, die eine Eisenverbindung und einen Katalysator enthält.

8. Batteriemodul (100) nach Anspruch 1, wobei:
der Neutralisatorgehäuseabschnitt (500) einen Kolben aufweist.

9. Batteriemodul (100) nach Anspruch 1, ferner aufweisend:
einen Neutralisatoreinspritzabschnitt (510), der in dem Neutralisatorgehäuseabschnitt (500) ausgebildet ist.

10. Batteriepack aufweisend das Batteriemodul (100) nach Anspruch 1.

## Revendications

1. Module de batterie (100) comprenant :
un empilement de cellules de batterie (120) dans lequel sont empilées plusieurs cellules de batterie (110),
un châssis de module (200) entourant l'empilement de cellules de batterie (120),
un cadre de barre omnibus (130) couvrant la partie de l'empilement de cellules de batterie (120) exposée depuis le châssis de module (200),
une barre omnibus (140) connectée à un conducteur d'électrode (111, 112) faisant saillie de l'empilement de cellules de batterie (120) à travers le cadre de barre omnibus (130),
un capteur de gaz (300) formé sur le cadre de barre omnibus (130),
et une partie de couvercle de barre omnibus (400) couvrant la barre omnibus (140),
**caractérisé en ce que** le capteur de gaz (300) est configuré pour détecter une fuite de sulfure d'hydrogène, et **en ce que** le module de batterie (100) comprend :
une partie de boîtier de l'agent neutralisant (500) formée sur la partie de couvercle de barre omnibus (400),
un agent neutralisant étant formé à l'intérieur de la partie du boîtier de l'agent neutralisant (500),
et la partie du boîtier de l'agent neutralisant (500) étant configurée pour être ouverte afin de refouler de l'agent neutralisant en réponse à la fuite.

2. Module de batterie (100) selon la revendication 1,
le capteur de gaz (300) étant formé afin d'être adjacent à la barre omnibus (140).

3. Module de batterie (100) selon la revendication 2,
la barre omnibus (140) comprenant une barre omnibus à bornes (141), et
le capteur de gaz (300) étant formé de façon à être adjacent à la borne (141a) de la barre de jonction (141).

4. Module de batterie (100) selon la revendication 3,
la partie de couvercle de barre omnibus (400) couvrant la borne (141a) de la barre de jonction (141).

5. Module de batterie (100) selon la revendication 1,
le module de batterie (100) comprenant en outre une flasque (150) couvrant le cadre de barre omnibus (130), et
comprenant en outre un capteur de gaz (310) formé sur la flasque (150).

6. Module de batterie (100) selon la revendication 1, comprenant en outre :
un capteur de gaz (320) formé entre une partie supérieure de l'empilement de cellules de batterie (120) et le châssis de module (200).

7. Module de batterie (100) selon la revendication 1,
l'agent neutralisant comprenant au moins un composant sélectionné dans le groupe comprenant un composé ferreux et un catalyseur.

8. Module de batterie (100) selon la revendication 1,
la partie de logement de l'agent neutralisant (500) comprenant un bulbe.

9. Module de batterie (100) selon la revendication 1, comprenant en outre :
une partie d'injection d'agent neutralisant (510) formé dans la partie de logement de l'agent neutralisant (500).

10. Bloc-batterie comprenant le module de batterie (100) selon la revendication 1.
